# EUROPEAN PATENT SPECIFICATION

(11) **EP 0 350 151 B1**
(45) Date of publication and mention of the grant of the patent: **30.03.1994**
(21) Application number: 89304188.9
(22) Date of filing: 26.04.1989
(51) Int. Cl.: A61K 31/70

(54) **Diagnosing and treating chronic fatigue syndrome**
Diagnose und Behandlung chronischer Ermüdungserscheinungen
Diagnostic et traitement du syndrome de la fatigue chronique

(30) Priority: 07.07.1988 US 220765
(43) Date of publication of application: 10.01.1990
(73) Proprietor: HEM PHARMACEUTICALS CORP., Rockville, MD 20852 (US)
(72) Inventor: Carter, William A., Birchrunville, PA 19421 (US)
(74) Representative: Hallybone, Huw George

(56) References cited:
- EP-A- 0 213 921
- EP-A- 0 285 263
- EP-A- 0 299 745
- EP-A- 0 306 347
- THE LANCET, 06 June 1987; W.A. CARTER et al., pp. 1286-1292#
- PROCEEDINGS OF THE 24TH ANNUAL MEETING OF THE AMERICAN SOCIETY OF CLINICAL ONCOLOGY, New Orleans, LA (US), 22-24 May 1988, vol. 7; D.R. STRAYER et al., p. 2, no. 6#
- PROCEEDINGS OF THE 23RD ANNUAL MEETING OF THE AMERICAN SOCIETY OF CLINICAL ONCOLOGY, Atlanta, GA (US), 17-19 May 1987, vol. 6; W.A. CARTER et al., no. 8#
- JOURNAL OF BIOLOGICAL RESPONSE MODIFIERS, vol. 4, 1984, Raven Press, New York, NY (US); I. BRODSKY et al., pp. 669-675#
- AIDS RESEARCH, vol. 2, no. 2, 1986, Mary Ann Liebert Inc. (Publishers); J.M. WU et al., pp. 127-131#
- NUCLEIC ACIDS RESEARCH, vol. 9, no. 7, 1981, IRL Press Ltd., London (GB); D.H. WRESCHNER et al., pp. 1571-1581#
- CELL, vol. 43, 1985; J.N. ZULLO et al., pp. 793-800#
- EUROPEAN JOURNAL OF BIOCHEMISTRY, vol. 143, 1984; P.J. CLAVEY et al., pp. 165-174#
- ANNALS OF INTERNAL MEDICINE, vol. 116, 1992; pp. 102-113#

## Description

### BACKGROUND OF THE INVENTION

Chronic Fatigue Syndrome (CFS), a condition involving some 10 to 12 million Americans, is a difficult to diagnose, ubiquitous disorder characterized by extreme fatigue, lymph gland enlargement and constitutional symptoms such as weight loss, loss of appetite and the like. The condition occurs primarily in young, active people. While some CFS patients manifest neuropsychiatric changes such as depression, loss of memory and similar derangements, chronic fatigue syndrome is sometimes difficult to distinguish from entirely neurological disorders, particularly depression. Various laboratory studies indicate that many different viruses may replicate in individuals having Chronic Fatigue Syndrome, and that these individuals become, in effect, "virus sewers". Viruses such as Epstein-Barr, cytomegalovirus, retroviruses, herpes viruses, etc., are often present in such individuals.

It has been determined that a specific deficiency in 2′-5′A molecules exists in individuals having Chronic Fatigue Syndrome, which deficiency has diagnostic significance of enormous value. As an illustration, consider that many 25-30 year old women with very active small children at home often complain of "chronic fatigue", but are not necessarily virus-manufacturing facilities. The diagnostic procedures here described enable the clinician to ascertain which patients presenting symptoms of chronic fatigue and related symptoms including in some instances loss of weight, loss of appetite and neuropsychiatric changes, are properly classified as having Chronic Fatigue Syndrome with associated viral involvement and accurately distinguishing such patients from those presenting fatigue symptoms caused by other often external reasons and/or depression. Proper diagnosis of Chronic Fatigue Syndrome is the necessary prerequisite to effective therapy. These valuable diagnostic procedures are described below.

In addition to these diagnostic procedures, the first definitive therapy for this disorder has been developed using various double-stranded RNAs to correct the disorder and successfully treat the patient's condition.

In previous studies, the diagnostic utility of components of the 2′-5′ oligoadenylate/RNase L pathway, as described in more detail below has been reported, as it relates to viral disorders in general and retrovirus infections in particular (see e.g. EP-A-0 285 263). Specifically, it has now been determined that in Chronic Fatigue Syndrome, among other things, a previously unknown viral-associated inhibitor of RNase L is present coupled with an abnormally low level of 2′-5′A in the patient's lymphocytes. These two measurements act as indicators or "markers" for Chronic Fatigue Syndrome and thus can be used to definitively diagnose the syndrome in a highly reliable manner. Further, the diagnosis is conveniently conducted from a patient's blood sample outside the patient's body.

EP-A-0 213 921 discloses therapeutic methods of selectively inhibiting human viruses, specifically the AIDS virus (HIV), by administration of mismatched dsRNA. The ability of mismatched dsRNA to restore the proper functioning of the natural lymphocyte antiviral dsRNA-dependent (2'-5' oligoadenylate/RNase L) pathway is disclosed in Lancet, June 6, 1987, pp1286 - 1292.

### DESCRIPTION OF THE INVENTION

This invention includes procedures for identifying Chronic Fatigue Syndrome, as evidenced by a viral-associated inhibitor of RNase L coupled with low level of 2′-5′A in the patient's peripheral blood lymphocytes, diagnostic procedures using this information to determine the presence of Chronic Fatigue Syndrome, therapeutic procedures for restoring the patient's 2′-5′A deficit such as by administering exogenous dsRNAs and improving the patient's clinical condition, therapeutic procedures for monitoring a Chronic Fatigue Syndrome patient's condition and gauging the degree of dsRNA replacement required on an individual basis, and therapeutic compositions for treating Chronic Fatigue Syndrome.

### Diagnostic Procedures-

The in vivo concentration of 2′-5′A molecules in normal individuals and subjects with Chronic Fatigue Syndrome is assessed as follows: Ethanol-soluble fractions of patient samples (Ficoll-Hypaque-purified peripheral blood lymphocytes) were analyzed for their 2'-5′A content in 2′-5′A core-cellulose assays (affinity chromatography) with poly U-{³²P} -pCp. In this assay, the ability of 2′-5′A-activated RNase L to hydrolyze poly(U) is used to determine the concentration of functional 2′-5′A.

Reference values were established by testing 15 normal subjects with no recent history of viral infections as evidenced by lack of fever, absence of constitutional symptoms, rashes, etc. Concentrations of their lymphocyte 2′-5′A levels were determined using calibration curves obtained with authentic 2′-5′A molecules. Normal individual reference values, expressed as nanomoles of 2′-5′A per gram of lymphocyte protein, are consistently within the range of 0.2 to 2.0.

Using the same assay method, ten patients exhibiting the usual symptoms of Chronic Fatigue Syndrome were tested and the results obtained were as follows:

| Subject Number | n moles 2′-5′A per gram lymphocyte protein |
|---|---|
| 1 | <0.08 |
| 2 | <0.05 |
| 3 | <0.05 |
| 4 | <0.05 |
| 5 | nd* |
| 6 | nd* |
| 7 | <0.01 |
| 8 | <0.01 |
| 9 | <0.01 |
| 10 | <0.08 |

| | |
|---|---|
| * not detectable | |

Patients with Chronic Fatigue Syndrome have generally below 0.1 and always below about 0.2 n moles of 2′-5′A per gram of lymphocyte protein. Definitive treatment of such individuals with Chronic Fatigue Syndrome is provided by supplying exogenous dsRNAs, as required, until the intracellular level of 2′-5′A oligonucleotides reaches normal and/or the patient's clinical symptomology abates. The patient's resistance to Chronic Fatigue Syndrome and opportunistic viruses is maintained by continuing to measure the patient's intracellular 2′-5′A oligonucleotide levels and supplying exogenous dsRNA, as required, to maintain the 2′-5′A level in the normal range, usually in excess of 0.2 nanomoles of 2′-5′A per gram of lymphocyte protein.

The natural dsRNAs play a role in host defense when challenged with a viral disease such as Chronic Fatigue Syndrome. Specific reduction in bioactive dsRNA, or enzymes which depend on dsRNA, notably a viral-associated inhibitor of RNase L coupled with abnormally low levels of 2′-5′A in peripheral blood lymphocytes, within specific cells contributes to disease progression. dsRNA, notably mismatched dsRNAs, reverse disease progression.

By "mismatched dsRNA" are meant those in which hydrogen bonding (base stacking) between the counterpart strands is relatively intact, i.e., is interrupted on average less than one base pair in every 29 consecutive base pair residues. The term "mismatched dsRNA" should be understood accordingly. The dsRNA may be a complex of a polyinosinate and a polycytidylate containing a proportion of uracil bases or guanidine bases, e.g., from 1 in 5 to 1 in 30 such bases
(poly I · poly(C₄₋₂₉x>U or G)).

The dsRNA may be of the general formula rIₙ·r(C₁₁₋₁₄,U)ₙ or rIₙ·r(C₁₂,U)ₙ. Other suitable examples of dsRNA are discussed below.

The mismatched dsRNAs preferred for use in the present invention are based on copolynucleotides selected from poly (Cₙ,U) and poly (Cₙ,G) in which n is an integer having a value of from 4 to 29 are mismatched analogs of complexes of polyriboinosinic and polyribocytidilic acids, formed by modifying rIₙ·rCₙ to incorporate unpaired bases (uracil or guanidine) along the polyribocytidylate (rCₙ) strand. Alternatively, the dsRNA may be derived from poly(I)·poly(C) dsRNA by modifying the ribosyl backbone of polyriboinosinic acid (rIₙ), e.g., by including 2′-O-methyl ribosyl residues. The mismatched complexes may be complexed with an RNA-stabilizing polymer such as lysine and cellulose. These mismatched analogs of rIₙ·rC_{n′} preferred ones of which are of the general formula rIₙ·(C₁₁₋₁₄,U)ₙ or rIₙ1·r(C₂₉,G)ₙ, are described by Carter and Ts'o in U.S. Patents 4,130,641 and 4,024,222. The dsRNAs described therein generally are suitable for use according to the present invention.

Other examples of mismatched dsRNA for use in the invention include: -
poly (I) · poly (C₄,U)
poly (I) · poly (C₇, U)
poly (I) · poly (C₁₃,U)
poly (I) · poly (C₂₂,U)
poly (I) · poly (C₂₀,G)
poly (I) · poly (C₂₉,G) and
poly (I) · poly Cₚ₂₃ G>p
2′-5′A concentration and molecular size may be quantitated by high pressure liquid chromatography (HPLC). Ribosomal RNA cleavage assays may be used to assess biological functionality (activity) of the 2′-5′A-synthesized by the patient in vivo or to determine the level of activated RNase L in patient samples. Peripheral mononuclear blood cells are the preferred cells for analysis.

Patients having Chronic Fatigue Syndrome are treated with intravenous infusions of 200 to 250 mg of rI·r(C₁₁₋₁₄,U) twice weekly and 2′-5′A levels increase in association with clinical improvement. The amount of dsRNA administered and the frequency of administration will be guided by the 2′-5′A levels measured in conjunction with the patient's clinical improvement. Amounts of dsRNA administered will provide a level of from 0.01 to 1,000 micrograms of dsRNA per milliliter of the patient's systemic blood circulation immediately following administration measured at a point distal from the point of infusion.

## Claims

1. A method of diagnosing chronic fatigue syndrome in a human patient comprising assessing the level of intracellular 2′-5′A oligonucleotides in a sample of the patient's peripheral blood and comparing same to predetermined levels of 2′-5′A oligonucleotides in healthy individuals, reduced 2′-5′A oligonucleotide levels as compared with those in healthy individuals indicating the presence of chronic fatigue syndrome.

2. A method of distinguishing viral-associated chronic fatigue syndrome from primary psychological or neuropsychiatric disorders resembling same in a person comprising assessing the level of intracellular 2′-5′A oligonucleotides in a sample of the patient's peripheral blood and comparing same to predetermined levels of 2′-5′ oligonucleotides in healthy individuals.

3. The method of claim 1 or claim 2 in which the 2′-5′A oligonucleotide is 2′-5′ oligoadenylate.

4. The method of claim 1 or claim 2 in which the diagnosis is presumptively positive for chronic fatigue syndrome when the 2′-5′ oligonucleotide in the patient sample is less than 0.2 nanomoles of 2′-5′A per gram of lymphocyte protein.

5. Use of a mismatched dsRNA for the manufacture of a medicament for treating viral-associated chronic fatigue syndrome.

6. Use of claim 5 in which the mismatched dRNA is polyadenylic acid complexed with polyuridylic acid.

7. Use of claim 5 in which the mismatched dsRNA is a complex of a polyinosinate and a polycytidylate containing from 1 in 5 to 1 in 30 uracil or guanidine bases.

8. Use of claim 7 in which the mismatched dsRNA is rIₙ·r(C₁₁₋₁₄,U)ₙ or the mismatched dsRNA contains regions of bond breakage and exhibits the favorable therapeutic ratio property of rIₙ·r(C₁₁₋₁₄,U)ₙ.

## Patentansprüche

1. Verfahren zur Diagnose des Chronischen Müdigkeitssyndroms (Chronic Fatigue Syndrome CFS) bei einem menschlichen Patienten, bei welchem Verfahren der Gehalt an intrazellulären 2'-5'A Oligonucleotiden in einer Probe von peripherem Blut des Patienten festgestellt und mit vorherbestimmten Gehalten von 2'-5'A Oligonucleotiden in gesunden Wesen verglichen wird, wobei reduzierte Gehalte von 2'-5'A Oligonucleotiden im Vergleich zu jenen in gesunden Wesen auf das Vorliegen von Chronischem Müdigkeitssyndrom hinweisen.

2. Verfahren zur Unterscheidung des Virus-assoziierten Chronischen Müdigkeitssyndroms von primären psychologischen oder neuropsychiatrischen Störungen, die demselben ähnlich sind, an einer Person, bei welchem Verfahren der Gehalt an intrazellulären 2'-5'A Oligonucleotiden in einer Probe von peripherem Blut des Patienten festgestellt und mit vorherbestimmten Gehalten von 2'-5'A Oligonucleotiden in gesunden Wesen verglichen wird.

3. Verfahren nach Anspruch 1 oder 2, bei welchem das 2'-5'A Oligonucleotid ein 2'-5'-Oligoadenylat ist.

4. Verfahren nach Anspruch 1 oder 2, bei welchem die Diagnose von Chronischem Müdigkeitssyndrom presumptiv positiv ist , wenn das 2'-5' Oligonucleotid in der Patientenprobe weniger als 0,2 Nanomol 2'-5'A pro Gramm Lymphocytenprotein beträgt.

5. Verwendung von mismatched dsRNA für die Herstellung eines Medikaments zur Behandlung des Virus-assoziierten Chronischen Müdigkeitssyndroms.

6. Verwendung nach Anspruch 5, bei welcher die mismatched dRNA Polyadenylsäure im Komplex mit Polyuridylsäure ist.

7. Verwendung nach Anspruch 5, bei welcher die mismatched dsRNA ein Komplex eines Polyinosinats und eines Polycytidylats mit einem Gehalt von 1 in 5 bis 1 in 30 Uracil- oder Guanidinbasen ist.

8. Verwendung nach Anspruch 7, bei welcher die mismatched dsRNA rIₙ.r(C₁₁₋₁₄,U)ₙ ist oder die mismatched dsRNA Bindungsbruchbereiche enthält und die Eigenschaft des günstigen therapeutischen Verhältnisses von rIₙ.r(C₁₁₋₁₄,U)ₙ aufweist.

## Revendications

1. Procédé de diagnostic du syndrome de la fatigue chronique chez un malade humain dans lequel on évalue le niveau d'oligonucléotides 2'-5'A intracellulaires dans un échantillon du sang périphérique du malade et on le compare à des niveaux prédéterminés d'oligonucléotides 2'-5'A chez des individus en bonne santé, des niveaux d'oligonucléotides 2'-5'A réduits par comparaison avec ceux d'individus en bonne santé indiquant la présence du syndrome de fatigue chronique.

2. Procédé pour distinguer le syndrome de fatigue chronique associé à un virus d'avec des désordres psychologiques ou neuropsychiatriques primaires qui lui ressemblent chez une personne, dans lequel on évalue le niveau d'oligonucléotides 2'-5'A intracellulaires dans un échantillon du sang périphérique du malade et on le compare à des niveaux prédéterminés d'oligonucléotides 2'-5' chez des individus en bonne santé.

3. Procédé de la revendication 1 ou de la revendication 2 dans lequel l'oligonucléotide 2'-5'A est le 2'-5'-oligoadénylate.

4. Procédé de la revendication 1 ou de la revendication 2 dans lequel le diagnostic est présumé positif pour le syndrome de fatigue chronique lorsque l'oligonucléotide 2'-5' dans l'échantillon du malade est inférieur à 0,2 nanomole de 2'-5'A par gramme de protéine de lymphocyte.

5. Utilisation d'un ARN à deux brins mésapparié pour préparer un médicament pour le traitement du syndrome de fatigue chronique associé à un virus.

6. Utilisation selon la revendication 5, dans laquelle l'ARNd mésapparié est de l'acide polyadénylique complexé avec de l'acide polyuridylique.

7. Utilisation selon la revendication 5, dans laquelle l'ARN à deux brins mésapparié est un complexe d'un polyinosinate et d'un polycytidylate contenant des bases uracile ou guanidine dans un rapport compris entre 1 sur 5 et 1 sur 30.

8. Utilisation selon la revendication 7, dans laquelle l'ARN à deux brins mésapparié est rIₙ.r(C₁₁₋₁₄,U)ₙ ou l'ARN à deux brins mésapparié contient des régions de rupture de liaison et présente la propriété de rapport thérapeutique favorable de rIₙ.r(C₁₁₋₁₄,U)ₙ.
